# EUROPÄISCHE PATENTSCHRIFT

(11) **EP 2 346 997 B1**
(45) Veröffentlichungstag und Bekanntmachung des Hinweises auf die Patenterteilung: **26.02.2014**
(21) Anmeldenummer: 09775929.4
(22) Anmeldetag: 30.06.2009
(51) Int. Cl.: C12P 5/02, C02F 3/28, C12M 1/107

(54) **Verfahren zur Herstellung von Methan aus Prozesswässern und biogenem Material**
Method for producing methane from process water and biogenic material
Procédé de production de méthane à partir d'eaux industrielles et d'un matériau biogène

(30) Priorität: 10.07.2008 DE 102008032409
(43) Veröffentlichungstag der Anmeldung: 27.07.2011
(73) Patentinhaber: Rietzler, Johann, 90475 Nürnberg (DE)
(72) Erfinder: Rietzler, Johann, 90475 Nürnberg (DE)
(74) Vertreter: Neidl-Stippler, Cornelia
(86) Internationale Anmeldenummer: PCT/DE2009/000932
(87) Internationale Veröffentlichungsnummer: WO 2010/003397

(56) Entgegenhaltungen:
- EP-A- 1 065 268
- EP-A- 1 790 732
- WO-A-2006/021087
- DE-A1- 10 327 954
- DE-A1-102005 047 719
- US-A- 4 311 593
- US-A1- 2002 148 778

## Beschreibung

Die Erfindung betrifft ein Verfahren zur Herstellung von Methan nach Patentanspruch 1 sowie eine Biogasanlage nach Patentanspruch 7.

Die Vergärung von biologischen Stoffen ist seit langem bekannt. Aufgrund von verschiedensten Entwicklungen wurden ein-, zwei- oder mehrstufige Verfahren entwickelt. Neben der aus der Güllevergärung entwickelten Nassvergärung wird ebenfalls die Trockenvergärung praktiziert. Diese Verfahren wurden vielfach in die Praxis umgesetzt. Ein Verfahren zur Herstellung von Methan aus organischen bioverfügbaren Inhaltsstoffen von Abwässern ist aus der DE 10 2004 053 615 A1 bekannt geworden. Es verwendet einen Perkolator als Hydrolysestufe und einen Bioreaktor mit Methanbakterien für die Biogasherstellung. Dadurch, dass die Perkolatorflüssigkeit in einen Speicher gebracht wird, kann diese dann, falls neues Gas benötigt wird, in den Bioreaktor zur Gasproduktion verbracht werden. Dieses Verfahren ist verbesserungsfähig, da es bei großen Ab- und Prozeßwassermengen nicht die notwendigen Kapazitäten zur Verfügung stellen kann. Die US2002/148778 A1 lehrt ein Verfahren zum Vergären von pflanzlichen Rohstoffen mit hoher Kohlenhydratkonzentration zu Biogas. WO2006/021087A1 beschäftigt sich mit der Verwertung von Resten der Alkoholdestillation ohne Zusatz weiterer pflanzlicher Rohstoffe zu Biogas. DE 10 2005 047719 A1 beschreibt die Biogasproduktion aus Biomasse. US 411593 lehrt die Verwertung von Abwasser der Molasseproduktion in einer Biogasanlage. Diese bekannten Verfahren liefern aber keine Lösung für die Zeiten, in denen keine Molasseproduktion bzw. Alkoholdestillation erfolgt.

Dann, wenn Vegetationsruhe besteht oder kein Prozeßwasser zur Verfügung steht, können die bekannten Verfahren mangels notwendiger Speicherkapazität nicht mehrere Monate überbrücken. Falls Methanbakterien aber unversorgt bleiben, sterben sie ab. Ferner ist dann keine Biogasproduktion möglich.

Der Erfindung liegt die Aufgabe zugrunde, große Ab- Prozesswassermengen, insbesondere aus der landwirtschaftlichen Produktion, wie der Zucker- und Ethanol-Produktion, die bisher unbehandelt auf die Nutzflächen verteilt wurden, einer bedarfsgerechten Nutzung zuzuführen. Da die Ab- Prozesswassermengen nicht ganzjährig zur Verfügung stehen, soll in den Zeiten eines Produktionsstillstandes von Zucker und Ethanol in Verbindung mit Biomasse ein ganzjähriger Betrieb der Biogaserzeugung ermöglicht werden.

Die Aufgabe wird durch ein Verfahren mit den Merkmalen des Patentanspruches 1 sowie eine Biogasanlage mit den Merkmalen des Patentanspruches 7 gelöst. Vorteilhafte Ausgestaltungen ergeben sich aus den abhängigen Ansprüchen

Dieses Verfahren hat den Vorteil, dass es auf einer einfachen Technik zum Abbau von biogenem Material basiert, das bisher in der Regel unbehandelt auf den Anbauflächen verteilt wurde.

Erfindungsgemäss ist somit ein Abbau von Ab- Prozesswässern und Nebenprodukten aus der Herstellung von Zucker und Ethanol und biogenem Material mit ganzjährigem Betrieb des Biogasreaktors möglich, mit dem periodisch anfallende Ab- und Prozesswässer in großen anfallenden Mengen von über 10.000 m³/Tag aufbereitet werden und in den übrigen Perioden durch den Einsatz von Frischwasser und Kreislaufführung des Substratwassers in Verbindung mit nachwachsenden Rohstoffen oder organischem Material eine ganzjährige Biogasnutzung mit energetischer Verwertung des Biogas möglich ist.
Das gereinigte Abwasser wird ebenso wie der anfallende Schlamm für Beregnungs- und Dünge zwecke bereitgestellt.

Durch Nutzung von Frischwasser und Kreislaufführung von Substratwasser zum Ausgleich von Lieferschwankungen und zur Vergleichmäßigung des Biogasbetriebes kann eine Rückführung des Substratwassers sowie von Rückführschlämmen in/oder zwischen den Vorbehälter und die Biogasreaktoren vorgesehen sein. Das hier anfallende Wasser kann ebenso für Bewässerungszwecke genutzt werden

So wird eine bedarfsgerechte und ganzjährige Bewässerung sowie eine kontinuierlich Steuerung des Biogasanfalls ermöglicht und der Biogasbedarf, zum Beispiel für die Verströmung oder Wärmeerzeugung, dauerhaft bzw. in Spitzen- bzw. Schwachlastzeiten entsprechend geregelt werden. Während bei bekannten Anlagen die Steuerung der Biogasproduktion nicht oder nur in engeren Perioden zur Verbrauchsanpassung erfolgt, kann mit dem erfindungsgemäßen Abbauverfahren eine Vergleichmäßigung der Biogasnutzung und Produktion von Strom und Wärme erfolgen.

Als biogenes Material werden alle von Lebewesen, wie Pflanzen, Tieren, Einzellern, Viren etc. stammenden Substanzen angesehen, insbesondere können dies Schlempe/Ab- und Prozesswässer und weitere natürliche Nebenprodukte der Produktion von nachwachsenden Rohstoffen, wie: Waschwasser, Fuselöle und Filterkuchen oder Zuckerrohrblätter aus der Ethanol- und Zuckergewinnung, Bioabfalle, Grunschnitt, Gewerbeabfalle, Lebensmittelabfalle, landwirtschaftliche Abfalle, nachwachsende Rohstoffe, Fermenterlaugen, Abwasser der Stärkeproduktion aus Kartoffeln, Erbsen und Bohnen od. dgl. und ahnliche Stoffe sein.

Bevorzugt werden Ab-/Prozesswässer sowie das biogene Material und die o.g. Nebenprodukte in einem entsprechend dimensionierten Puffer-Reaktions bzw. Vorbehälter (z.B. 24 h Puffer) fur Flussigkeiten in einem ausgewählten Verhältnis zueinander gespeichert, da die durch Bakterien durchgeführten Methanreaktionen im anschliessenden anaerob betriebenen Biogasreaktor fur die Biogasproduktion im Stundenbereich liegen. Methanbakterien sind thermisch empfindlich, daher sollten die für den jeweiligen Bakterienstamm möglichen Maximaltemperaturen, bspw. ca. 55°C, besser auch ca. 37°C in den Methangasreaktoren nicht überschritten werden.

In einer bevorzugten Ausführungsform wird unter Prozesstemperaturen von bis zu ca. 95 °C anfallendes Ab- und Prozesswasser bzw. auf ca. 55 °C temperiertes Ab- Prozesswasser oder Substrat- oder Frischwasser mit Biomasse bzw. biologisch abbaubaren nachwachsenden Rohstoffen oder Nebenprodukten wie Fuselöle und (Zuckerrohr)-Filterkuchen sowie Waschwasser in einem Behälter vermischt und die entstehende Maische nach einer Reaktionszeit von bis zu 24 Std. unter Abzug des entstehenden Kohlendioxids in den Sohlbereich einer Lagunenanlage flächenhaft eingetragen. Durch die homogene Verteilung der Maische sowie eine Zirkulation von Biogas innerhalb des Biogasreaktoren bei gleich bleibenden Teinperaturbedingungen von ca. 55 °C +./. 2 °C und ca. 37 °C wird ein optimaler Prozess der kontinuierlichen Biogasentwicklung unter Abbau des organischen Kohlenstoffs in den genannten thermophilen und mesophilen Bereichen erreicht mit einem weitest-gehender Abbau von biogenem Material innerhalb von 7 bis 15 Tagen. Durch den Pro-zess der Biogaszirkulation mit dem Substrat erfolgt neben dem Vermischungsvorgang des Substrats eine Minderung der Kohlenstoffdioxid-Anteile im Biogas ebenso wie durch den mesophilen Betrieb mit relativer Er-höhung der Methangasanteile und Erhöhung des Heizwertes. Die Kohlenstoffdioxid-Anteile können durch Zugabe von Kalkmilch oder alkalischen Waschwässern weiter reduziert werden. Da die Prozesswässer nicht ganzjährig in gleicher Menge zur Verfügung stehen, werden die Lagunenbehälter modulartig mit mehreren Becken betrieben, die mit Leitungen miteinander verbunden sind.

Nachfolgend wird die Erfindung anhand von Prozeßwasser der Ethanol/Zuckerproduktion, auch Vinhaca genannt, erläutert.

Anfallendes Ab-Prozesswasser z.B. der Ethanol- Zucker-Produktion hat aus der Destille heraus eine Temperatur von bis zu 95 °C. Bei Vermischung mit kleingehäckselten pflanzlichen Rohstoffen in einem belüfteten Reaktionsbehälter wird die Blattstruktur der Pflanze nachhaltig zerstört, so dass eine rasche Bioverfügbarkeit abzubauender Stoffe in dem anschliessenden anaerob betriebenen Biogasreaktor erfolgen kann. Dadurch kann der Abbauprozess von pflanzlichen Rohstoffen, der in herkömmlichen Biogasreaktoren bis zu 53 Tage beträgt, nachhaltig verkürzt werden. Durch Anwendung eines Wärmetauschers erfolgt eine Temperaturerniedrigung der im Reaktionsbehälter befindlichen Flüssigkeit auf ca. 55 - 58 °C vor der Einbringung in den mindestens einen anaerob betriebenen Biogasreaktor. Bei den genannten Temperaturen erfolgt im Reaktionsbehälter beschleunigte Hydrolyse und Säurebildung unter CO2-Entwicklung statt, wobei das CO2 unter den im Reaktionsbehälter vorliegenden aeroben Bedingungen teilweise durch Luftzufuhr ausgetrieben werden kann. Bei einem verminderten Angebot von Ab-Prozesswasser wird Frisch- oder Substratwasser bei ca. 52 -57 °C mit den gehäckselten pflanzlichen Rohstoffen und den o.g. Nebenprodukten vermischt, eingesetzt.

Nach einer Aufenthaltszeit von max. 24 Std. im Reaktionsbehälter wird die Maische einem Biogasreaktor in Lagunenform zugeführt, in dem unter anaeroben Bedingungen die eigentliche Methanogenese mittels Methanbakterien mit Bildung von Methan und Kohlenstoffdioxid statt-findet. Die methanbakterien können an Trägern immobilisiert oder aber frei sein. Der/die Lagunenbehälter sind mit einem Tragluftdach versehen, wobei der darunter befindliche freie Raum als Gasspei-cher dient. Bevorzugt wird das im Gasspeicherraum befindliche Biogas teilweise in den Sohlbereich des Lagunenbehälters eingepresst, dadurch eine bessere Zirkulation des Substrats sowie Abführung des Methan und bessere Biogasentwicklung durch Entfernung der Hemmung des Reaktionsgleichgewichts erreicht. Ferner kann Alkali, wie Kalkmilch, zur weiteren Bindung von Kohlenstoffdioxid in die Lagunenbehälter zudosiert werden. Die Lagunenanlage kann aus mindestens 2 durch Leitungen miteinander ver-bundenen Becken bestehen, die dem unterschiedlichen Angebot von bis zu über 10.000 m³/Tag anfallenden Ab-/Prozesswässern gerecht werden. Der Reaktor wird durch das beschriebene Wärmetauschersystem dauerhaft auf Temperaturen von ca. 55 °C und/oder ca. 37 °C gehalten. Durch Zirkulation von Biogas in den Lagunenbehältern findet trotz der Vermischung des Substrates ein Absetzvorgang von Sink- und/oder Schwimmstoffen statt. Diese werden im Sohlbereich über Schneckenpumpen aus dem Reaktor gefördert und nach Passage von Siebbandpressen oder vergleichbaren Entwässerungseinrichtungen wie Zentrifugen für Düngezwecke bereitgestellt und das Filtrations- und/oder Substratwasser zwischen den Reaktionsbehälter und der Lagunenanlage rückgespült. Die Lagunenbehälter weisen darüber hinaus einen Überlauf aus, über den das entgaste Substrat-Wasser entschlammt, abgeleitet und für Düngezwecke bereitgestellt oder als Ersatz von Ab-/Prozesswasser rückgeführt wird.

Das Vergären von Biogas fuhrt man bevorzugt mittels Bakterien durch. Dabei fuhrt man bevorzugt die Vergarung unter Beteiligung einer Bakterienmatrix aus mehreren Bakterienstammen durch. Je nach Bakterienstamm vermögen diese unterschiedliche Materialien zu vergären und liefern auch ein anderes Verhältnis von Methan/CO2. Der Biogasreaktor ist durch das oben beschriebene Wärmetauschersystem beheizbar, insbe-sondere außenbeheizbar. Damit kann im Biogasreaktor immer eine konstante Temperatur gehalten werden. Diese liegt bevorzugt bei ca. 55 und ca. 37° C, Die Verwendung des Eingabesystems zum Einbringen von biologisch abbaubaren Stoffen in den Biogasreaktor und die Rückführung von Substratwasser hat weiterhin den Vorteil, dass ein dauerhafter Betrieb der Biogasanlage jederzeit moglich ist. Außerdem ist durch den modularen Aufbau eine Anpassung an den jeweiligen Stoffanfall bzw. die Energieabnahme moglich Beim Abbau des bio-genen Materiais entstehen Sauren, daher sind die Mischbehälter sowie der Biogasreaktor bevorzugt saurefest.

Es ist ebenfalls denkbar, dass das Verfahren für andere Prozesse mit dem Anfallen von extrem sauren Prozesswässern im Biogasreaktor angewandt werden kann. Beispielsweise fällt in der Konservenindustrie einerseits ein hoher Abwasserstrom mit organischen Belastungen an, der meist stark sauer ist, andererseits gibt es aber auch feste Abfalle. Mit dem bedarfsgerechten Einsatz von festen biogenen und Pflanzlichen Rohstoffen in Verbindung mit dem Zuführen von Frischwasser und der Kreislaufführung von Substratwasser kann der jahreszeitlich schwankende Anfall von saurem Abwasser ausgeglichen werden, so dass in Zeiten geringen Abwasseranfalls immer noch eine gute Biogasproduktion vorhanden ist.

Der Biogasreaktor kann gasdicht sein und funktioniert bevorzugt nach einem der aus der Abwassertechnik ublichen Reaktorprinzipen (UASB- [Upflow anaerobic sludge bed],

Biogas besteht aus Methan (CH4) [50-85 Vol-%], Kohlendioxid (C02) [15-50 Vol-%] sowie Spuren von Sauerstoff, Stickstoff und Spurengasen (u.a. Schwefelwasserstoff). Mit dem erfindungsgemäßen mikrobiologischen Abbauverfahren wird Biogas mit einem hohen Methananteil von zwischen 60 und 80 Vol-% erzeugt. Es kann u.a. direkt fur Heizzwecke oder mittels eines Blockheizkraftwerks zur gekoppelten Produktion von Strom und Wärme genutzt werden. Die Erzeugung des Gases erfolgt durch anaerobe Vergärung organischer Stoffe. Zur Erhöhung des Biogasertrags kommen häufig Co-Fermentate zum Einsatz (zum Beispiel nachwachsende Rohstoffe oder Abfalle aus der Lebensmittelindustrie). Das vergorene organische Material kann anschließend als hochwertiger Dünger landbaulich verwertet werden.

Gemäß der Erfindung ist der Speicherpuffer bzw. der Misch/oder Vorbehalter belüftbar. Durch Mischung mit Luft kann Biogas leicht zu explosiven Gemischen führen, daher unterliegt die Herstellung und Speicherung besonderen Sicherheitsvorschriften.

Der Mischbehälter (2) hat bevorzugt ein Volumen von ca. 50 bis 100 % des täglich anfallenden Ab- Prozesswassers oder des Frischwassers. Er dient zum Vermischen der genannten zugeführten Materialströme (Flüssigkeiten mit Biomasse und Nebenprodukten aus der Zuckerrohrverarbeitung) und ist mit einem Wärmetauschersystem (7) versehen. Ferner kann eine Belüftung (6) für das Austreiben von Kohlendioxid vorgesehen werden. Die Biomasse wird durch einen Häcksler (3) feinfaserig zerkleinert und über ein Fördersystem (4) dem Mischbehälter zugeführt.

Eine solche Explosionsgefahr kann durch das erfindungsgemaße Verfahren ausgeschlossen werden, da eine Biogasproduktion ausschliesslich in der Lagunenanlage stattfindet, die mit einem Tragluftdach versehen ist, das gleichzeitig als Gasspeicherraum dient und der Betrieb weiterhin unter anaeroben Bedingungen erfolgt. Des weiteren ist eine Speicherung von Biogas nicht notwendig, da das gesamte Biogas unmittelbar in das anschließende BHKW überführt wird. Als Sicherheitssystem wird eine Notfackel installiert, die im Falle eines Ausfalles des BHKW in Betrieb gesetzt wird.

Nachfolgend wird die Erfindung anhand der Figuren und einer Zuckerrohrverarbeitungseinheit näher beschrieben, wobei sie keinesfalls auf die beispielhaft dargestellten Verfahren und Vorrichtungen eingeschränkt ist. Im einzelnen zeigt
Fig. 1 eine schematische Darstellung einer erfindungsgemäßen einfachen Biogasanlage (1) zur bedarfsgerechten Herstellung von Biogas aus Zuckerrohrabfällen; und
Fig. 2 eine schematische Darstellung einer weiteren erfindungsgemäßen Biogasanlage 81) mit mehreren Biogasreaktoren

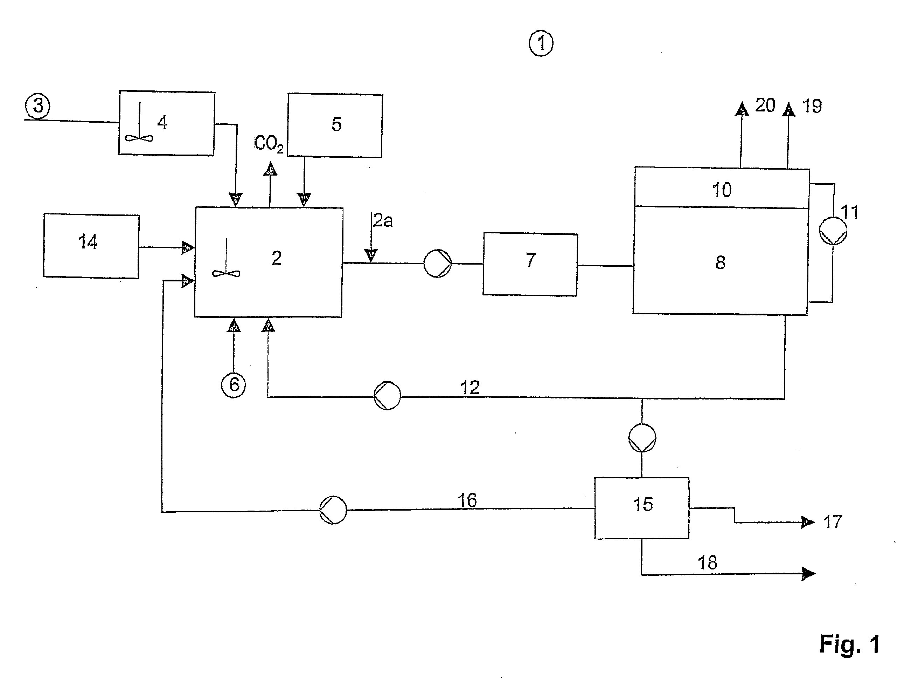

Wie in Fig. 1 gezeigt, wird Biomaterial aus einem Häxler 3 in ein Fördersystem 4, überführt. Aus dem Fördersystem 4 wird dieses zerkleinerte Biomaterial bedarfsweise in den Reaktions- und Mischbehälter 2 überführt. Ferner kann dem Mischbehälter Frisch/Waschlauge 12 - je nach Bedarf - zugeführt werden sowie Vinhaca (organische Reste aufweisende Flüssigkeit aus der Ethanoldestillation von vergorenem Zuckerrohr - ca 3 - 10 % organische Stoffe, und 1 % mineralische Feststoffe, Rest Wasser - etwa 4 - 5 Gew.% Trockenstoffe). Schließlich kann über eine Rückführleitung 12,16 Filtratwasser des Methanreaktors in den Reaktions- und Mischbehälter geführt werden.

Bei frischer Vinhaca aus der Produktion herrscht im Vinhaca-Bunker 5 eine Temperatur von bis zu 95°C sowie ein pH-Wert im Bereich des Sauren (ca 4 - 5.0 pH).
Die Waschlauge oder Waschwasser besitzt einen pH-Wert im stärker alkalischen, bevorzugt um pH 10 -12. Im Behälter 2 werden nun diese Materialströme zu einer Maische gemischt und - bevorzugt automatisch - auf einen schwach sauren pH Wert von um ca 5 eingestellt.

Dazu kann ein (nicht gezeigter) pH-Wert-Fühler im Reaktor 2 vorgesehen sein. Die so genannte Maische wird über einen Wärmetauscher 7 auf eine geeignete Temperatur für die im Biogasreaktor 8 anwesenden Methanbakterien gebracht. Ggf. kann der pH-Wert nochmals bei 2a gemessen und nachgeregelt werden.

Im Biogasreaktor 8 befinden sich freie oder aber immobilisierte Methanbakterien. Diese zersetzen Inhaltsstoffe der wässrige Lösung mit bis zu ca 12 Gew.% Trockenstoffen zu CO2 und Methangas. Das Methan wird im Gasspeicherraum 10 gesammelt und über die Leitung 19 abgeführt. Für überschüssiges Methan ist ein Notauslauf 20, der bspw. in einem Pufferbehälter oder einer Notfackel enden kann, vorgesehen.

Vom Gasspeicherraum führt eine Rezirkulationsleitung 11 zum Methanreaktorboden, um durch die Rückführung von Gas die Reaktion zu Methan zu begünstigen und reaktionshemmendes CO2 auszutreiben.

Von der in Umsetzung befindlichen Biogasreaktorlösung kann ein Anteil in den Vor/Mischbehälter 2 rückgeführt werden.

Die umgesetzte Bioreaktorlösung kann über die Schlammentwässerung in Filtratwasser 16, welches wieder in den Mischer 2 rückgeführt oder anderweitig (18)verwertet wird, abgezogen werden, wobei der Schlamm weiterer Verwendung zugeführt werden kann.

Dadurch, daß ständig Biomaterial vom Häcksler 3 über das Fördersystem 4 in den Reaktor 2 verbracht wird, ist ein Aufrechterhalten der Methangasbildung auch bei Fehlen von Vinhaca wegen Stillstands der Vinhaca-Produktion - möglich. Dies ist insbesondere deshalb wichtig, damit die Methanbakterien am Leben erhalten werden und eine weitere Produktion von Biogas möglich ist.

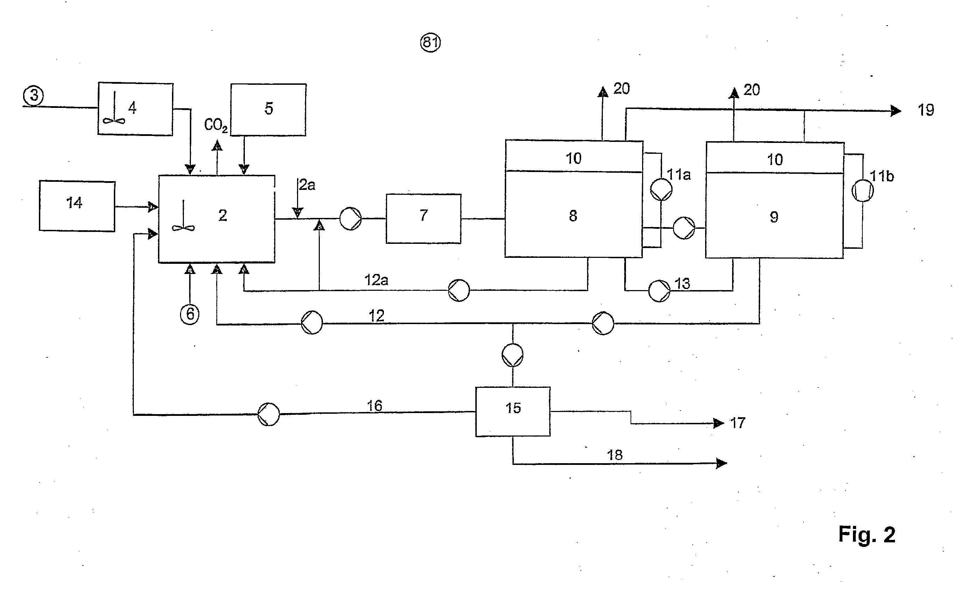

In Fig.2 ist eine aufwendigere Anlage für die Biogasherstellung aus den gleichen Ausgangsmaterialien, wie bei Fig. 1 erläutert, gezeigt.

Es werden dabei in Reaktor 2 wieder Biomaterial aus einem Häxler 3 und einem Aufgabebunker 4, Vinhaca aus der Quelle 45 und Waschlauge oder Frichwasser 14 in einem derartigen Verhältnis zugeführt, daß der Trockenmaterialgehalt der wässrigen Aufschlämmung bei etwa 12% liegt. Ferner wird nun Luftzufuhr in den Reaktor 2 vorgesehen, um CO2 aus der Maische auszutreiben, welches die Methanbildungsreaktion hemmt. Es sind auch Rückführleitungen 12, 12 a aus den Biogasreaktoren 8 und 9 zum Reaktor 2 vorgesehen. Die Maische wird sodann mittels eines geeigneten Wärmetauschers auf für die Methanbakterien um Biogasreaktor 8 geeignete bis optimale Temperatur und pH-Wert gebracht und dann in den Biogasreaktor 8 überführt. In diesem befinden sich Methanbakterien, die im Temperaturbereich um die 55° C arbeiten. Der Rückstand aus dem Methanbioreaktor 8 wird in einen weiteren, in Serie geschalteten Bioreaktor 9 überführt, in welchem bspw. ein etwas anderer Stamm Methanbakterien gehalten wird, der ggf. bei anderen Temperaturen um die 37° Carbeitet und ein etwas anderes Profil der Verarbeitung besitzt.

Die Biogasanlage 1 kann erfindungsgemäß insbesondere derart gefahren werden, dass der aerobe Mischbehälter und der anaerobe Biogasproduktionskreislauf strikt voneinander getrennt sind. Damit ist sichergestellt, dass keine sicherheitsrelevante Menge an freiem Biogas (Methan) vorliegt. Dies fuhrt zu einer verbesserten Betriebssicherheit der gesamten Anlage.

Während spezielle Ausführungsformen der Erfindung gezeigt und beschrieben wurden, sind vielfältige Abweichungen und alternative Ausführungsformen dem Fachmann offensichtlich. Demzufolge soll die Erfindung nur durch den Schutzumfang der Ansprüche begrenzt sein.

### Bezugszeichenliste

- 1: Biogasanlage
- 2: Vor/Mischbehälter
- 2a: pH-Wert-Steuerung
- 3: Häcksler
- 4: Fördersystem
- 5: Zugabe Vinhaca
- 6: Frischluftzuführung
- 7: Wärmetauscher
- 8: Biogasreaktor
- 9: Biogasreaktor
- 10: Speichergasraum
- 11: Biogaszirkulation
- 12: Substratwasserrückführung
- 13: Schlammrückführung mit Schneckenpumpe o.ä.
- 14: Frischwasser/Waschwassereingabe
- 15: Schlammentwässerung
- 16: Filtratwasserrückführung
- 17: Filterkuchen
- 18: Filtratwasser
- 19: Strom-/Dampferzeuger
- 20: Speicher-Pufferbehälter/Notfackel

## Patentansprüche

1. Verfahren zur Herstellung von Methan aus
I) Ab- und Prozesswasser aus der Herstellung von Zucker und Ethanol und
II) gehäxelten pflanzlichen Rohstoffen, wobei man:
- mindestens einen Misch/Vorbehälter (2) mit Ab- und Prozeßwasser und gehäxelten pflanzlichen Rohstoffen sowie ggf. Wasch- und/oder Frischwasser und/oder rückgeführtem Substratwasser unter Herstellung von Maische unter aeroben Bedingungen zur Hydrolyse im schwach Sauren bei pH von ca 5 beschickt,
- die Maische auf geeigneten pH-Wert und Temperatur einstellt;
- die so eingestellte Maische in mindestens einen anaerobe Methanbakterien aufweisenden Bioreaktor (8, 9) überführt und dort zur Biogasproduktion beläßt,
- das so entwickelte Biogas gewinnt, und
- die biologisch abgebaute Flüssigkeit abzieht.

2. Verfahren nach Anspruch 1, **dadurch gekennzeichnet, daß** Biogas in den/die Bioreaktor(en) (8, 9) rezirkuliert wird.

3. Verfahren nach einem der vorangehenden Ansprüche, **dadurch gekennzeichnet, daß** in den Bioreaktor Alkali ausgewählt aus der Gruppe bestehend aus: alkalische Lösung, alkalisches Waschwasser aus der Zucker- Ethanolproduktion, Kalkmilch, Ammoniak, zur weiteren pH-Wert-Erhöhung unter Erniedrigung des CO2-Anteils im entwickelten Biogas eingebracht wird.

4. Verfahren nach einem der vorangehenden Ansprüche, wobei durch Belüftung im MischNorbehälter (2) aerobe Verhältnisse eingestellt werden und Kohlenstoffdioxid ausgetrieben wird.

5. Verfahren nach einem der Ansprüche 1 bis 3, **dadurch gekennzeichnet, dass** im Misch/Vorbehälter (2) Normaldruck herrscht.

6. Verfahren nach einem der Ansprüche 1 bis 5, **dadurch gekennzeichnet, daß** das Vergären zu Biogas mittels zugesetzter und/oder immobilisierter Bakterien erfolgt.

7. Biogasanlage (1) für ein Verfahren nach einem der vorhergehenden Ansprüche, mit
mindestens einem mit einem Wärmetauschersystem (7) versehenen Misch-/Vorbehälter (2) für Maische aus gehäxelten pflanzlichen Rohstoffen und Ab- und Prozesswasser aus der Herstellung von Zucker und Ethanol, und
mindestens einem nachgeschalteten Biogasreaktor mit Biogas produzierenden Bakterien, wobei
- der Misch-/Vorbehälter (2) belüftbar ist; sowie
- ein System zur Zirkulation von Biogas im Substrat des Biogasreaktors;
- ein System zur Abtrennung von Schlämmen und ausgegorenem Substrat und
- Rückführung von Substrat- oder Filtratwasser (16) zum Biogasreaktor vorgesehen ist.

## Claims

1. Method for producing methane from waste and process water from the production of sugar and ethanol and chopped plant materials, wherein:
- you load at least one mix / pre-hopper (2) with waste and process water and chopped plant materials and possibly washing and / or fresh water and / or re-circulated substrate water by the production of mash under aerobic conditions for hydrolysis in the weak acidic at a pH of approximately 5,
- you set the mash to a suitable pH value and temperature,
- you transfer the mash set in this way to at least one anaerobic bioreactor (8, 9) having methane bacteria and keep it there for biogas production,
- you obtain the biogas developed in this way, and
- subtract the biologically decomposed liquid.

2. Method according to claim 1, **characterised in that** biogas is re-circulated in the bioreactor(s) (8, 9).

3. Method according to one of the previous claims **characterised in that** alkali was selected for the bioreactor from a group consisting of: alkaline solution, alkaline washing water and the sugar - ethanol production, lime wash, ammonia, introduced for further increasing the pH value and lowering the CO2 portion in the developed biogas.

4. Method according to one of the previous claims where aerobic conditions are set by ventilation in the mix / pre-hopper (2) and carbon dioxide is driven out.

5. Method according to one of the claims 1 to 3, **characterised in that** there is normal pressure in the mix / pre-hopper (2).

6. Method according to one of the claims 1 to 5, **characterised in that** the fermentation to biogas takes place using added and / or immobilised bacteria.

7. Biogas plant (1) for a method according to one of the previous claims, with
at least one mix / pre-hopper (2) furnished with a heat exchanger system (7) for mash made of chopped plant material and waste and process water from the production of sugar and ethanol, and
at least one downstream biogas reactor with biogas producing bacteria, in which the following is intended:
the mix / pre-hopper (2) can be vented; as well as a system for the circulation of biogas in the substrate of the biogas reactor;
a system for the separation of slurries and digested substrate and the recirculation of substrate or filtrate water (16) to the biogas reactor.

## Revendications

1. Méthode de production pour méthane à partir d'eaux usagées et de traitement provenant de production de sucre et éthanol et de matériaux végétaux hachés, où :
- charger au moins une trémie de mélange/préparation (2) avec des eaux usagées et de traitement et des matériaux végétaux hachés et peut-être aussi de l'eau de lavage et/ou de l'eau fraîche et/ou de l'eau de substrat recirculé par la production de moût sous des conditions aérobies pour une hydrolyse dans un acide faible d'environ 5 pH,
- amener le moût à une valeur de pH et une température adéquates,
- transférer le moût de façon à avoir au moins un bioréacteur anaérobique (8, 9) avec des bactéries de méthane et le réserver pour la production de biogaz,
- le biogaz est obtenu de cette façon, puis
- enlever le liquide biologiquement décomposé.

2. Méthode selon revendication 1, **caractérisée en ce que** le biogaz est recirculé dans le(s) bioréacteur(s) (8, 9).

3. Méthode selon une de nos revendications antérieures, **caractérisée en ce que** des alcalins ont été choisis pour le bioréacteur à partir d'un groupe contenant : solution alcaline, eau de lavage alcaline et la production sucre-éthanol, peinture à la chaux, ammoniac, introduit afin d'augmenter la valeur de pH et diminuer la part de CO2 dans le biogaz produit.

4. Méthode selon une de nos revendications antérieures, où les conditions aérobies sont réglées par une ventilation de la trémie de mélange/préparation (2) et le dioxyde de carbone est éliminé.

5. Méthode selon une de nos revendications 1 à 3, **caractérisée en ce que** la pression dans la trémie de mélange/préparation (2) est normale.

6. Méthode selon une de nos revendications 1 à 5, **caractérisée en ce que** la fermentation de biogaz se fait par addition ou immobilisation de bactéries.

7. Plante à biogaz (1), pour une méthode selon une de nos revendications antérieures, avec
au moins une trémie de mélange/préparation (2) munie d'un système d'échange de chaleur (7) pour un moût fait de matériaux végétaux hachés et d'eau usagée et de traitement de la production de sucre et d'éthanol, ainsi qu'
au moins un réacteur de biogaz en aval avec des bactéries de production de biogaz, dans lequel est prévu ce qui suit :
la trémie de mélange/préparation (2) peut être aérée, ainsi qu'un système pour la circulation du biogaz dans le substrat du réacteur de biogaz ;
un système de séparation des bouillies et du substrat digéré et la recirculation d'eau de substrat ou de filtrage (16) au réacteur de biogaz.
